# EUROPÄISCHE PATENTSCHRIFT

(11) **EP 1 667 608 B1**
(45) Veröffentlichungstag und Bekanntmachung des Hinweises auf die Patenterteilung: **05.06.2013**
(21) Anmeldenummer: 04786790.8
(22) Anmeldetag: 16.09.2004
(51) Int. Cl.: A61F 2/06, A61F 2/90

(54) **STENT MIT ENDSTÄNDIGEN VERANKERUNGSELEMENTEN**
STENT COMPRISING TERMINAL ANCHORING ELEMENTS
STENT COMPORTANT DES ELEMENTS D'ANCRAGE TERMINAUX

(30) Priorität: 16.09.2003 DE 10342757
(43) Veröffentlichungstag der Anmeldung: 14.06.2006
(73) Patentinhaber: pfm medical ag, 50966 Köln (DE)
(72) Erfinder: JUNG, Johannes, 76229 Karlsruhe (DE)
(74) Vertreter: Hohendorf, Carsten
(86) Internationale Anmeldenummer: PCT/DE2004/002071
(87) Internationale Veröffentlichungsnummer: WO 2005/027789

(56) Entgegenhaltungen:
- EP-A- 0 778 011
- EP-A- 1 121 911
- WO-A1-96/39102
- US-A1- 2002 198 587

## Beschreibung

Die Erfindung betrifft einen Stent zum Einsetzen in röhrenförmige Hohlorgane, insbesondere des menschlichen Körpers.

Gattungsgemäße Stents und ihre Verwendung sind an sich bekannt und werden zur Erweiterung und Offenhaltung röhrenförmiger Hohlorgane in diese eingesetzt. Derartige Stents weisen eine gitter- oder spiralförmige Struktur auf, die aus Wandsegmenten besteht. Zwischen und innerhalb der Wandsegmente werden Gitteröffnungen gebildet, die es ermöglichen, dass diese Struktur an ihrem Implantationsort in das Gewebe einwachsen kann. Solche Stents sind an sich bekannt und beispielsweise in der DE-A 197 46 882 oder in der WO 03/063 733 beschrieben.

Es ist bereits versucht worden, Stents derart auszugestalten, dass diese ihre Lage nach der Implantation nicht mehr verändern. So beschreibt beispielsweise die EP-A 0 778 011 die Ausbildung von verdickten Stentenden, wobei die nach außen vorstehenden, verdickten Enden nach Implantation vom Gewebe der Gefäßwand umwachsen werden, wodurch eine Lageveränderung, d. h. eine Verschiebung entlang der Röhrenachse verhindert werden soll. Die DE-A 197 46 882 beschreibt einen Stent, dessen röhrenförmige Gitterstruktur aus elastischen Wandsegmenten gebildet wird, die zickzack- oder V-förmig elastisch ausgebildet sind und deren spitze Enden - bezogen auf die Röhrenachse - in Radialrichtung nach außen aufgeweitet sind und so eine Lagestabilität auch in stärkeren Gefäßkrümmungen verhindern sollen.

Aus der WO 96/39102 ist ein Stent bekannt, welcher radial expandierbar ist, um eine bessere Fixierung in einem Gefäß zu erzielen. Zur Expansion des Stents wird eine externe Kraft aufgebracht oder ein Rückhalteelement in einem selbstexpandierbaren Stent entfernt. Da der Stent über seine gesamte Länge expandiert, kann er nur bedingt im Bereich bewegter und/oder starker seitlicher Abzweigungen, sowie im herznahen Bereich verwendet werden.

Die EP 1 121 911 A2, von welcher der Oberbegriff des Anspruchs 1 ausgeht, offenbart ein Stent-Implantat zur Einfügung an einem Zielort in einem Gefäß eines Patienten. Das Implantat weist einen gefalteten Zustand zum Zuführen zum Zielort und einen erweiterten Zustand zur Implantation an diesem auf. Das Implantat umfasst einen selbsterweiternden äußeren Stent, der ein röhrenförmiges Element ist, das aus einem superelastischen Material hergestellt ist. Das Implantat umfasst ferner ein röhrenförmiges flexibles poröses Implantatelement, das sich entlang der Innenseite des äußeren Stents erstreckt. Das Implantatelement umfasst ein vorderes und ein hinteres Ende, die über das vordere und hintere Ende des äußeren Stents gefaltet sind, um Manschetten zu bilden. Das Implantat umfasst ferner einen selbsterweitemden inneren Stent, der ebenfalls ein röhrenförmiges Element ist, das aus einem superelastischen Material hergestellt ist. Der innere Stent ist innerhalb der Innenseite des Implantatelements so angeordnet, dass sowohl der innere Stent als auch das Implantatelement und der äußeren Stent aneinander liegen. Der offenbarte Stent weist jedoch über seine Länge eine im wesentlichen gleichbleibende Flexibilität auf, woraus sich die zuvor zu dem Stent aus der WO 96/39102 genannten Nachteile ergeben.

Die Erfindung hat zum Ziel, einen Stent, insbesonders einen expandierbaren Stent bereitzustellen, bei dem nicht nur die Lagestabilität sichergestellt ist, sondern der insbesonders im herznahen Bereich, sowie im Bereich bewegter und/oder starker seitlicher Abzweigungen verwendet werden kann, ohne die Gefahr, einen Blutfluss zur Abzweigung zu behindern.

Dieses Ziel wird mit den in den Ansprüchen definierten Merkmalen erreicht.

Es wurde nämlich gefunden, dass die zuvor genannten Ziele sich dadurch lösen lassen, dass bei einem Stent der gattungsgemäßen Art am röhrenförmigen Stentende bogenförmige Verankerungselemente angeordnet werden, die mit den endständigen Wandsegmente bildenden Wandelementen einstückig verbunden sind. Dabei werden vorzugsweise nicht benachbarte Elemente brückenförmig verbunden. Das Verankerungselement weist an seiner Bogenspitze einen größeren radialen Abstand zur Stentachse auf, als die endständigen Wandelemente, so dass bezogen auf den Durchmesser oder das Lumen des röhrenförmigen Stents das Ende nach außen hin aufgeweitet ist, und die Enden oder Spitzen der Bögen sich klemm- bzw. krallenartig in die Organwand am Implantationsort verankern.

Erfindungsgemäß ist das bogenförmige Verankerungselement zu seiner Spitze verlaufend nach außen hin kurvenförmig gekrümmt, wobei die Krümmung zur Bogenspitze hin zunimmt und die Bogenspitze einen röntgenopaken Bereich aufweist und wobei am Röhrenende an mindestens dem endständigen sowie dem axial davor liegenden nächsten Wandsegment jedes Wandelement des endständigen Segmentes mit seinem axial davor liegenden Element des nächsten Segmentes mittels einem Verbindungssegment verbunden ist.

Die Verankerungselemente sind ebenso wie die Wandsegmente aus Stegen gebildet und weisen vorzugsweise eine größere Breite und/oder Dicke auf, als die Stege der Wandelemente. Die Form der bogenförmigen Verankerungselemente kann sowohl rein kreisbogenförmig als auch oval oder ovalspitz ausgestaltet sein. Vorzugsweise ist der Bogen jedoch als Spitzbogen V-förmig ausgebildet. In einer ganz besonders bevorzugten Ausführungsform verläuft der Spitzbogen in einer leicht s-förmigen Kurve. An der Bogenspitze ist das Verankerungselement vorzugsweise verstärkt und ist in einer besonders bevorzugten Ausführungsform in diesem Bereich radioopak ausgestaltet. Auf diese Weise kann der behandelnde Arzt genau die Endpunkte und Lage des Stentes während des Eingriffes verfolgen. Die Enden oder Füße des bogen- oder V-förmigen Verankerungselementes sind mit den Wandelementen des Stents einstückig verbunden, und sind weder angeschweißt oder verklebt, noch mittels einer anderen Verbindungstechnik an den Stent angebracht.

In einer besonderen Ausführungsform überbrückt der Bogen des Verankerungselementes zwei nicht benachbarte Wandelemente eines endständigen, ringförmigen Wandsegmentes. Üblicherweise liegen zwischen den überbrückten Wandelementen mindestens ein, vorzugsweise zwei weitere Elemente. Im Endbereich des Stentes sind die Wandsegmente anders als im mittleren Teil direkt ohne Federelemente miteinander verbunden, so dass diese entlang der Röhrenachsrichtung nicht oder nur in begrenztem Umfange streckbar sind. Auf diese Weise wird dem Endbereich des Stentes trotz Flexibilität eine für die Verankerung notwendige, ausreichende Festigkeit verliehen.

Die Ausbildung der Wandsegmente in dem zwischen den Stentenden liegenden Bereich kann bei der erfindungsgemäßen Ausführung beliebig ausgestaltet werden. Derartige flexiblen Wandsegmente sind dem Fachmann bekannt und beispielsweise in der DE-A 100 50 940, der DE-A 197 46 882 und anderweitig beschrieben.

Vorzugsweise wird der erfindungsgemäße Stent aus elastischen ringförmigen Wandsegmenten gebildet, die entlang einer longitudinalen Achse angeordnet sind und so eine schlauchförmige Gitterwand bilden. Durch seine Elastizität kann der Stent den Bewegungen des Hohlorgans folgen, in das er implantiert ist. Die einzelnen Wandsegmente sind mittels eines durchgehenden Längsstegs miteinander verbunden. In einer besonderen Ausführungsform ist der Längssteg linear durchgehend ausgebildet und kann so Druckspannungen oder Zugspannungen in Längsrichtung aufnehmen, ohne dass dadurch eine Längenänderung des Stents bewirkt wird. Gleichermäßen bewirkt ein Kontrahieren, also ein Zusammendrücken der einzelnen Wandsegmente ebenfalls keine Längenänderung des Stents, da die dabei eventuell auftretenden Zugspannungen oder Druckspannungen auf den durchgehenden Längssteg übertragen und von diesem aufgenommen werden.

Eine Weiterbildung der Erfindung zeichnet sich dadurch aus, dass die ringförmigen Wandsegmente Wandelemente umfassen bzw. aus einer Mehrzahl solcher Elemente gebildet sind. Diese Wandelemente sind vorzugsweise Federelemente die wechselweise unter einem aus ersten Federelementen und zweiten Federelementen gebildeten Winkel zueinander angeordnet sind. Hierdurch ergibt sich für die Wandsegmente eine zickzackartige elastische Struktur, wodurch eine gute, elastische Federwirkung erreicht wird, die auch eine zur Stentachse radiale Expansion ermöglicht und die Stützwirkung auf das Hohlorgan bewirkt.

Die Federelemente können in etwa geradlinig ausgebildet sein. Diese geradlinige Ausbildung ist im Bezug auf eine Projektion auf eine Außenumfangsfläche des Stents zu verstehen.

Die Verbindungssegmente verbinden zweckmäßigerweise entweder nur erste oder zweite Federelemente miteinander. Die Verbindungssegmente selbst sind gegenüber entlang der Stentlängsachse einwirkende Schub- und Zugkräften nicht elastisch und im wesentlichen starr, d. h. dass diese zusammen mit den durch sie verbundenen Federelementen diese Zug- und Schubkräfte aufnehmen und eine Längenänderung des Stents verhindern. Dadurch bilden die jeweils verbundenen ersten bzw. die zweiten Federelemente gemeinsam mit den Verbindungssegmenten den durchgehenden Längssteg. Zu diesem Zweck sollten die Verbindungssegmente und die damit verbundenen Federelemente jeweils parallel zu einander angeordnet sein. Dies gilt wieder im Bezug auf die Projektion auf eine Umfangsfläche des Stents. Auf diese Weise wird gewährleistet, dass die Krafteinleitung nur entlang des Längsstegs wirkt und keine Querkomponente hat, die zu einer unerwünschten Verkürzung oder Verlängerung des Stents führen könnte. Dies schießt eine longitudinale Expansion oder Kontraktion ermöglichende nicht linearen Verlauf aus, wie er z. B. durch einen zickzackförmigen oder wellenförmigen Verlauf des Längssteges entsteht.

Eine Ausführungsform der Erfindung zeichnet sich durch mehrere in einer Projektion auf eine Außenumfangsfläche zueinander parallele Längsstege aus, die in Umf angsrichtung voneinander beabstandet angeordnet sind. Dies können beispielsweise drei oder vier Längsstege sein. Auf diese Weise werden die Wandsegmente besonders wirkungsvoll zueinander positioniert, wobei gleichzeitig eine Längenänderung des Stents zuverlässig vermieden wird.

Es ist auch möglich, dass der Längssteg eine schraubenlinienartige Gestalt hat. Dies kann beispielsweise dann der Fall sein, wenn der Längssteg aus den Verbindungssegmenten und den ersten bzw. zweiten Federelementen besteht. Dies führt zu einem besonders einfachen Aufbau. Längenänderungen aufgrund von Druckspannungen oder Zugspannungen oder durch ein Zusammendrücken des Stents werden trotzdem zuverlässig vermieden.

Die Verbindungssegmente können auch eine größere Materialdicke bzw. Breite aufweisen als die Federelemente. Insbesondere dann, wenn ein selbstexpandierender Stent mittels Laser aus einem röhrenförmigen Körper mit geringem Durchmesser geschnitten wird, ergibt sich ein geringer Aufwand. In dem ersten Zustand mit geringem Durchmesser sind die Verbindungssegmente dann beispielsweise in etwa S-förmig ausgebildet. In dem zweiten Zustand mit größerem Durchmesser weisen die Verbindungssegmente dann zumindest eine Komponente parallel zu dem Längssteg auf. Die Verbindungssegmente können beispielsweise die doppelte Breite der Federelemente haben. Dadurch ergibt sich ein besonders einfaches Schnittmuster.

Vorzugsweise ist der Stent einstückig ausgeführt. Auf diese Weise ergibt sich eine stabile Konstruktion ohne unnötige Kanten oder Sollbruchstellen.

Als Material für den Stent kann ein Formgedächtnis-Material, wie beispielsweise ein sogenanntes Memory-Metall, nämlich eine Nickel-Titan-Legierung, wie sie auch unter dem Namen Nitinol vertrieben wird, verwendet werden. Auch Polymere, wie sie in anderen Bereichen der Medizin zur Implantation im Körper verwendet werden, sind zur Herstellung des erfindungsgemäßen Stents geeignet. Aus einem röhrenförmigen Körper mit geringem Durchmesser kann dann beispielsweise mittels Laserstrahlung ein geeignetes Schnittmuster zum Bereitstellen der Federelemente und der Verbindungselemente herausgeschnitten werden. Auf bekannte Weise kann diesem röhrenförmigen Körper dann die expandierte Form aufgeprägt werden. Wird der so erzeugte Stent anschließend in den Zustand mit kleinem Durchmesser komprimiert und beispielsweise mittels eines Katheters in ein erkranktes Blutgefäß eingeführt, so kann der Stent an seiner Position durch Erwärmen über die sog. Konversionstemperatur wieder automatisch in die eingeprägte Form gebracht werden.

Als weitere Materialien für den Stent bieten sich außerdem Edelstahl, Kunststoff oder sog. selbstauflösende Materialien an. Insbesondere diese selbstauflösenden Materialien sind dann von Vorteil, wenn ein Stent nicht dauerhaft gelegt werden soll. Wenn keine selbstexpandierenden Stents verwendet werden, können diese an der gewünschten Position beispielsweise mittels eines Ballonkatheters expandiert werden.

Vorzugsweise sollte die Oberfläche des Stents bearbeitet, insbesondere veredelt, geglättet und/oder poliert sein. Auf diese Weise ergibt sich eine glatte und körperverträgliche Oberfläche.

Die Erfindung soll anhand der folgenden Figuren näher erläutert werden. Es zeigen:
Fig. 1 einen handelsüblichen Stent gemäß dem Stand der Technik.
Fig. 2 einen Stent mit erfindungsgemäßen Verankerungselementen.
Fig. 3 eine vergrößerte Teilansicht eines Stents von Fig. 2.
Fig. 4 zeigt eine für den erfindungsgemäßen Stent bevorzugte Gitternetzstruktur, wie sie für den mittleren Wandbereich des Stentes verwendet wird.

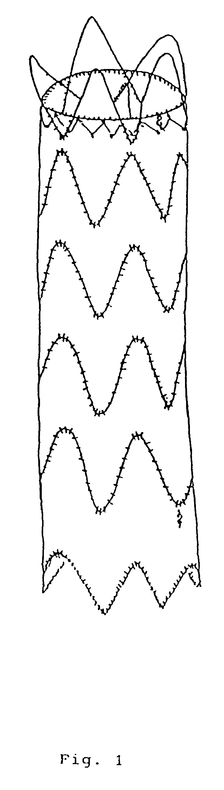

Bei einem Stent gemäß des Standes der Technik, wie er in Fig. 1 dargestellt ist, sind sinus-wellenförmige Wandsegmente auf einem zylindrischen, gewebeartigen Material aufgenäht. Dabei sind die wellenförmigen Wandelemente nur an der Nahtstelle des zylinderförmigen Gewebes als Steg miteinander verbunden. Am oberen Ende weist der Stent ein aufgenähtes, offenes Drahtsegment auf, welches wie die wellenförmigen Wandsegmente gebildet ist, jedoch genau gegenläufig, d. h. um eine halbe Phase verschoben hierzu verläuft ohne dass sich das offene Endsegment mit den Wandsegmenten berührt. Zur Verstärkung ist der obere Rand des Gewebematerials mittels einem zickzack verlaufenden Drahtfaden verstärkt.

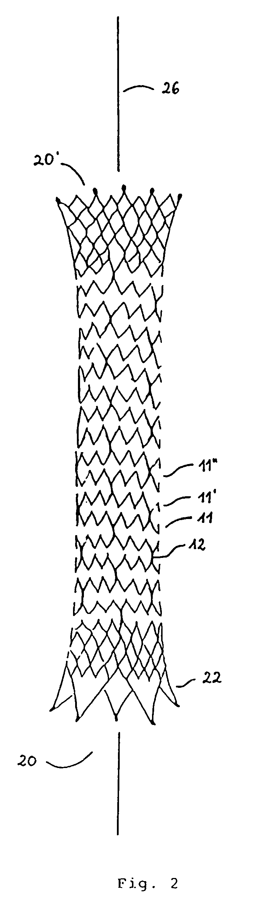

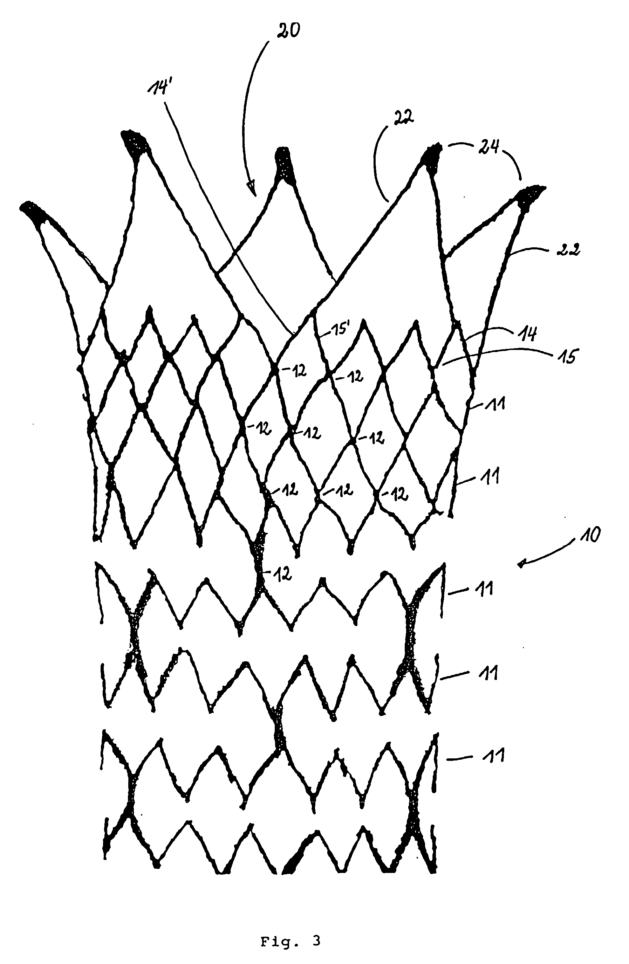

Fig. 2 zeigt einen erfindungsgemäßen Stent, dessen gitterförmige Röhrenwand mittel einem üblichen Schnittmuster ausgestaltet ist, wie es beispielsweise in der DE-A 197 46 882 beschrieben ist. Der röhrenförmige Stent verläuft entlang einer Achse 26 und ist an seinen Enden 20, 20' nach außen hin aufgeweitet. Das in Fig. 3 zur besseren Darstellung vergrößerte Ende 20 des Stents von Fig. 2 zeigt den Aufbau der Wandsegmente 11, die aus V-förmigen Wandelementen 14, 15 aufgebaut sind, wobei die V-förmigen Elemente an ihren Spitzen miteinander verbunden sind und so eine zirkumferential um die Stentlängsachse 26 verlaufende Zickzacklinie bilden. Die einzelnen Wandelemente 14, 15 werden durch aus an ihrer Stegspitze miteinander verbundenen Federelementen 14 und 15 gebildet. Im Bereich des Stentendes sind die hintereinander liegenden, ringförmigen Wandsegmente 11 an jedem ihrer Wandelemente 14, 15 über Verbindungssegmente 12 miteinander verbunden, so dass ein rautenförmiges Gittemetz entsteht. Am Ende dieser Gitternetzstruktur sind die erfindungsgemäßen Verankerungselemente 22 derart angeordnet, dass sie mit der distalen Spitze der Wandelemente 14 und 15 einstückig verbunden sind. Dabei sind die bogen- oder V-förmigen Stege der Elemente 22 gegenüber den Wandelementen 14, 15 verbreitert. Dies ist durch eine einfache Schneidetechnik beim Zurechtschneiden des Stentes leicht herstellbar. Die Bogenspitze 24 des Verankerungselementes 22 ist mit einem radioopaken Material versehen, so dass die Lage vom durchführenden Arzt am Röntgenbild leicht erkennbar ist. Wie sich außerdem den Fig. 2 und 3 entnehmen lässt, sind die Verankerungselemente 22 nach außen aufgebogen, so dass ihre Spitze 24 einen größeren Abstand zur Stentachse 26 aufweist, als die Wand- bzw. Federelemente 14, 15, mit denen sie verbunden sind. Erfindungsgemäß weisen die Verankerungselemente 22 eine nach außen gerichtete Krümmung auf, wobei der Krümmungsradius zur Bogenspitze hinlaufend zunimmt. Da die Verankerungselemente 22 aus dem gleichen elastischen Material wie der Stent 10 selbst ausgebildet sind, entsteht durch diese Form eine Klemmspannung, welche die Spitzen 24 in das umgebende Organ drückt und somit den gesamten Stent 10 verankert.

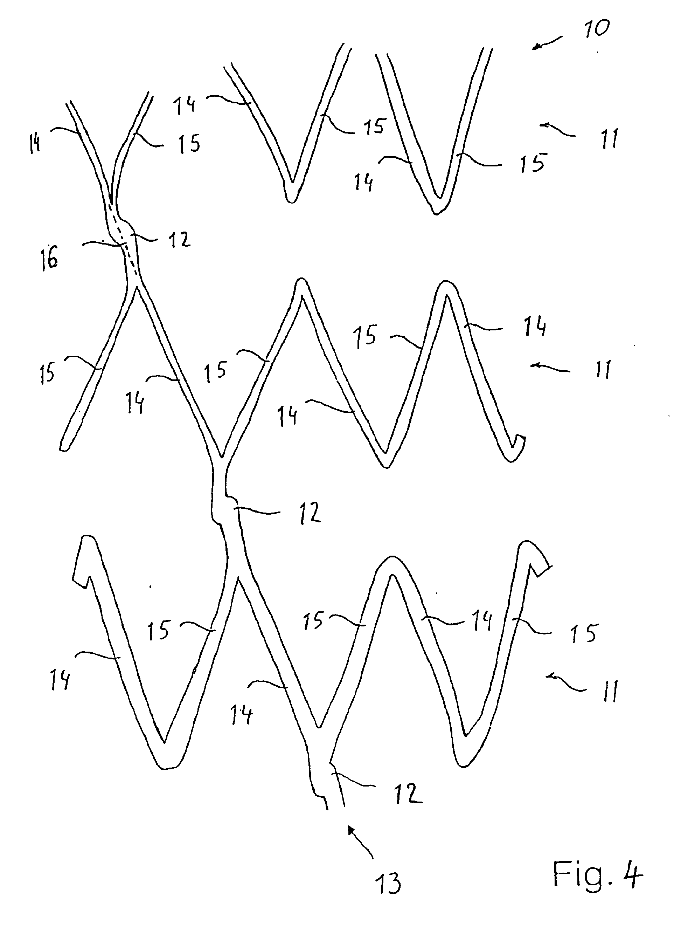

Fig. 4 zeigt nun eine bevorzugte Gitterstruktur, wie sie in der DE-102 43 136 sowie in parallelen Anmeldungen angegeben ist. Ein Stent 10 mit einem solchen Muster wird mittels Laserstrahlung aus einem Röhrchen aus einem geeigneten Formgedächtnis-Material, nämlich eines Memory-Metalls, wie z. B. Nitinol, ausgeschnitten. Wie sich der Figur entnehmen lässt, sind in einem ersten Zustand nach dem Einschneiden des Schnittmusters in das Röhrchen aus Memory-Metall die ersten Federelemente 14 und die zweiten Federelemente 15 einander benachbart angeordnet. Die ersten Federelemente 14 und die diesen benachbarten zweiten

Federelemente 15 sind dabei im geschnittenen, nicht expandierten Zustand parallel aneinander liegend angeordnet. Gut in der Figur zu sehen ist die in etwa S-förmige Gestalt der Verbindungssegmente 12. Außerdem sind einander benachbart angeordnete Wandsegmente 11 jeweils um einen Versatz gegeneinander versetzt angeordnet, welcher der Dicke des ersten Federelementes 14 und des zweiten Federelementes 15 entspricht. Auf diese Weise ist in dem ersten Zustand ein erstes Federelement 14 eines Wandsegmentes 11 an seinen Enden jeweils mittels Verbindungselementen 12 mit einem um den Versatz versetzten ersten Federelements 14 der benachbarten Wandsegmente 11 verbunden. Nach dem Einschneiden des Schnittmusters in einen röhrenförmigen Rohling z. B. aus Memory-Metall wird der so hergestellte Stent in einen zweiten Zustand expandiert, der einen größeren Durchmesser hat als der erste Zustand. Dieser zweite Zustand wird dem Stent 10 dann auf bekannte Weise aufgeprägt. Zur Implantation mittels eines Katheters wird der so vorbereitetet Stent 10 dann in einen Zustand mit geringem Durchmesser zusammengedrückt. Nach der gewünschten Positionierung kann der Stent 10 dann durch Erwärmen über die sogenannten Konversionstemperatur wieder in die eingeprägte Form des zweiten Zustands expandiert werden. Es ist aber auch möglich, den Stent 10 mittels eines Ballonkatheters zu expandieren.

### Bezugszeichenliste

| | |
|---|---|
| 10 | Stent |
| 11 | Wandsegment |
| 12 | Verbindungselement |
| 13 | Längssteg |
| 14 | erstes Federelement |
| 15 | zweites Federelement |
| 16 | Krafteinleitrichtung |
| 17 | Stent |
| 18 | Längssteg |
| 20 | Röhrenende |
| 22 | Verankerungselement |
| 24 | Bogenspitze |
| 26 | Röhrenachse |

## Patentansprüche

1. Stent (10) mit einer um eine longitudinale, biegsame Röhrenachse (26) verlaufende, aus einer flexiblen Gitterstruktur gebildeten röhrenförmigen Wand mit an entgegengesetzten Achsenenden liegenden Röhrenenden (20), wobei die Wand aus ringförmigen Wandsegmenten (11) gebildet ist, die entlang der Achse aneinandergereiht sind und die mittels Verbindungssegmenten (12) miteinander verbunden sind und wobei die ringförmigen Wandsegmente (11) Wandelemente (14, 15) mit einer elastischen Struktur umfassen, wobei die Wand an mindestens einem Röhrenende (20, 20') ein flexibles, bogenförmiges Verankerungselement (22) aufweist, welches mit mindestens zwei endständigen Wandelementen (14, 15, 14', 15') einstückig verbunden ist und mindestens ein elastisches Wandelement (14, 15) überbrückt und dass das bogenförmige Verankerungselement (22) an seiner Bogenspitze (24) einen größeren radialen Abstand zur Röhrenachse (26) aufweist, als die endständigen Wandelemente (14, 15) und zu seiner Spitze (24) verlaufend nach außen hin kurvenförmig gekrümmt ist,
**dadurch gekennzeichnet,**
**dass** die Krümmung zur Bogenspitze (24) hin zunimmt und die Bogenspitze (24) einen röntgenopaken Bereich aufweist, wobei am Röhrenende (20, 20') an mindestens dem endständigen sowie dem axial davor liegenden nächsten Wandsegment (11) jedes Wandelement (14, 15) des endständigen Segmentes (11) mit seinem axial davor liegenden Element (14, 15) des nächsten Segmentes (11) mittels einem Verbindungssegment (12) verbunden ist.

2. Stent nach Anspruch 1, **dadurch gekennzeichnet, dass** der Bogen des Verankerungselementes (22) V-förmig ausgebildet ist.

3. Stent nach Anspruch 1, **dadurch gekennzeichnet, dass** der Bogen des Verankerungselementes (22) eine größere Materialbreite aufweist, als die Wandelemente (14, 15).

4. Stent nach Anspruch 1, **dadurch gekennzeichnet, dass** das Verankerungselement mindestens zwei Wandelemente (14, 15) überbrückt.

5. Stent nach Anspruch 1, **dadurch gekennzeichnet, dass** er mindestens drei Verankerungselemente (22) aufweist.

6. Stent nach einem der vorhergehenden Ansprüche, **dadurch gekennzeichnet, dass** die Wandelemente (14, 15) der Wandsegmente (11) des gegenüberliegenden Röhrenendes (20) röntgenopake Bereiche (24) aufweisen.

7. Stent nach einem der vorhergehenden Ansprüche, **dadurch gekennzeichnet, dass** das gegenüberliegende Röhrenende (20') bezogen auf die Röhrenachse (26) radial aufgeweitet ist und einen größeren Durchmesser als die Stentmitte aufweist.

8. Stent nach einem der vorhergehenden Ansprüche, **dadurch gekennzeichnet, dass** die Aufweitung entlang der Röhrenachse (26) an mindestens zwei ringförmigen Wandsegmenten (11) vor dem Ende beginnt.

## Claims

1. A stent comprising a tubular wall that extends along a longitudinal, flexible tubular axis (26), is formed from a flexible grid structure, and has tube ends (20) lying on opposing axis ends, wherein the wall is formed by annular wall segments (11) that are lined up along the axis and are interconnected by means of connection segments (12), and wherein the annular wall segments (11) comprise wall elements (14, 15) having an elastic structure, wherein the wall comprises a flexible, arched anchoring element (22) on at least one tube end (20, 20'), which anchoring element is integrally connected with at least two terminal wall elements (14, 15, 14', 15') and bridges at least one elastic wall element (14, 15) and wherein the arched anchoring element (22) presents, at its curve tip (24), a larger radial distance from the tube axis (26) than the terminal wall elements (14, 15) and is bent outward in a curved shape in the direction of its tip (24),
**characterized in that**
the curvature increases in the direction of the curve tip (24) and the curve tip (24) comprises a radiopaque region, wherein at the tube end (20, 20') on at least the terminal wall segment (11) and the wall segment (11) axially disposed in front of it, each wall element (14, 15) of the terminal segment (11) is connected with its element (14, 15), axially disposed in front of it, of the next segment (11) by means of a connecting element (12).

2. A stent according to claim 1, **characterized in that** the curve of the anchoring element (22) is V-shaped.

3. A stent according to claim 1, **characterized in that** the curve of the anchoring element (22) has a larger material thickness than the wall elements (14, 15).

4. A stent according to claim 1, **characterized in that** the anchoring element bridges at least two wall elements (14, 15).

5. A stent according to claim 1, **characterized in that** it comprises at least three anchoring elements (22).

6. A stent according to one of the preceding claims, **characterized in that** the wall elements (14, 15) of the wall segments (11) of the opposite tube end (20) comprise radiopaque regions (24).

7. A stent according to one of the preceding claims, **characterized in that** the opposite tube end (20') is radially expanded with respect to the tubular axis (26) and has a larger diameter than the centre of the stent.

8. A stent according to one of the preceding claims, **characterized in that** the expansion along the tubular axis (26) begins at least two annular wall segments (11) before the end.

## Revendications

1. Stent (10) comprenant une paroi tubulaire qui s'étend autour d'un axe tubulaire flexible et longitudinal (26), qui est formée à partir d'une structure de réseau flexible et qui comprend des extrémités de tube (20) qui se trouvent à des extrémités d'axe opposées, la paroi étant formée par des segments de paroi (11) annulaires qui sont mis à la file le long de l'axe et qui sont reliés l'un à l'autre par moyen des éléments de liaison (12), et les segments de paroi annulaires (11) comprenant des éléments de paroi (14, 15) ayant une structure élastique, la paroi comprenant un élément d'ancrage (22) flexible arqué qui est relié d'un seul tenant à au moins deux éléments de paroi (14, 15, 14', 15') terminaux et superpose au moins un élément de paroi (14, 15) élastique et l'élément d'ancrage (22) arqué présentant, à sa pointe de l'arc, une plus grande distance radiale de l'axe de tube (26) que les éléments de paroi (14, 15) terminaux et étant courbé vers l'extérieur de manière incurvée dans la direction de sa pointe (24),
**caractérisé en ce que**
la courbure augmente dans la direction de la pointe de l'arc (24) et la pointe de l'arc (24) comprend une zone radiopaque, à l'extrémité du tube (20, 20') et sur au moins le segment de paroi terminal (11) et le segment de paroi (11) disposé axialement devant celui-ci, chaque élément de paroi (14, 15) du segment terminal (11) étant relié à son élément (14, 15) disposé axialement devant celui-ci du segment suivant (11) par moyen d'un élément de liaison (12).

2. Stent selon la revendication 1, **caractérisé en ce que** l'arc de l'élément d'ancrage (22) est en forme de V.

3. Stent selon la revendication 1, **caractérisé en ce que** l'arc de l'élément d'ancrage (22) a une plus grande épaisseur de matière que les éléments de paroi (14, 15).

4. Stent selon la revendication 1, **caractérisé en ce que** l'élément d'ancrage superpose au moins deux éléments de paroi (14, 15).

5. Stent selon la revendication 1, **caractérisé en ce qu'**il comprend au moins trois éléments d'ancrage (22).

6. Stent selon l'une des revendications précédentes, **caractérisé en ce que** les éléments de paroi (14, 15) des segments de paroi (11) de l'extrémité de tube opposée (20) comprennent des zones radiopaques (24).

7. Stent selon l'une des revendications précédentes, **caractérisé en ce que** l'extrémité de tube opposée (20') est radialement élargie par rapport à l'axe de tube (26) et a un plus grand diamètre que le centre du stent.

8. Stent selon l'une des revendications précédentes, **caractérisé en ce que** l'élargissement le long de l'axe de tube (26) commence au moins deux segments de paroi annulaires (11) avant la fin.
